# EUROPEAN PATENT APPLICATION

(11) **EP 4 403 242 A1**
(43) Date of publication of application: **24.07.2024**
(21) Application number: 21957484.5
(22) Date of filing: 15.09.2021
(51) Int. Cl.: B01D 53/04, B01D 53/72

(54) **ETHYLENE OXIDE GAS REMOVAL METHOD, AND ETHYLENE OXIDE GAS REMOVAL SYSTEM USING SAME**

(71) Applicant: TERUMO Kabushiki Kaisha, Tokyo 151-0072 (JP)
(72) Inventor: MORIHARA, Atsushi, Nagaokakyo-shi, Kyoto 617-0833 (JP); KATO, Yoshihiko, Nagaokakyo-shi, Kyoto 617-0833 (JP); KUZUOKA, Hiroki, Nagaokakyo-shi, Kyoto 617-0833 (JP); IMAMURA, Hiroshi, Nagaokakyo-shi, Kyoto 617-0833 (JP); HAYASHI, Kazuya, Fujinomiya-shi, Shizuoka 418-0015 (JP)
(74) Representative: Casalonga
(86) International application number: PCT/JP2021/033935
(87) International publication number: WO 2023/042302

(57) **Abstract**

An ethylene oxide gas removal method of the present invention is a method for removing ethylene oxide gas emitted into a predetermined partition space (12), and the method includes: a first step of suctioning EO-containing air (CA) that contains ethylene oxide emitted into the partition space (12) and supplying the EO-containing air (CA) to a concentration device (14); a second step of removing a water content from the suctioned EO-containing air (CA), and thereafter adsorbing and concentrating ethylene oxide by an adsorbing material (42), in the concentration device (14); a third step of desorbing the concentrated ethylene oxide from the adsorbing material (42) and sending the ethylene oxide to a removal device (16); and a fourth step of decomposing and removing ethylene oxide gas by the removal device (16).

## Description

### Technical Field

The present invention relates to a method for removing ethylene oxide gas that exists in a predetermined space at an extremely low concentration, and a device (removal system) for removing the ethylene oxide gas.

### Background Art

Ethylene oxide (hereinafter, may be abbreviated as "EO") has extremely high reactivity, and is thus used as an intermediate when another organic substance is synthesized. Furthermore, ethylene oxide has high sterilizability, and is thus used also for sterilizing medical instruments and precision equipment, for example.

Among the above-described usages, for example, in sterilization of a medical instrument, after ethylene oxide has been used for the sterilization, the used ethylene oxide has been previously emitted into the atmosphere in an unprocessed state in some cases. However, ethylene oxide may become a human carcinogen and cause human skin/mucosa irritation. In addition thereto, in recent years, influence of ethylene oxide emitted into the atmosphere on the environment has caused significant issues. Therefore, regulation concerning ethylene oxide is stipulated in both Japan and foreign countries.

As a technique for removing the used ethylene oxide, for example, Patent Literature 1 (Japanese Laid-Open Patent Publication No. 2014-237090) discloses a waste gas processing device for removing ethylene oxide which is contained in waste gas after the sterilization, and the waste gas processing device includes an adsorption cylinder that is filled with activated carbon in which petroleum or coal is a raw material, allows the waste gas to come into contact with the activated carbon, and adsorbs and decomposes ethylene oxide to remove ethylene oxide from the waste gas.

Furthermore, Patent Literature 2 (Japanese Laid-Open Patent Publication No. H08-215541) discloses a processing device in which an adsorption tower for adsorbing ethylene oxide in exhaust gas, and a catalytic combustion device for combusting ethylene oxide in exhaust gas are connected in series.

These techniques can provide a processing device and a processing method that have excellent efficiency in removing EO that is contained in waste gas after the sterilization.

### Citation List

### [Patent Literature]

[PTL 1] Japanese Laid-Open Patent Publication No. 2014-237090
[PTL 2] Japanese Laid-Open Patent Publication No. H08-215541

### Summary of Invention

### Technical Problem

However, the above-described conventional techniques have the following problems. That is, the above-described conventional processing device and processing method are effective for removing gas containing EO at a relatively high concentration such as waste gas emitted from an EO sterilizer of a medical instrument. However, in a case where a small amount of EO is emitted into a predetermined partition space, it is extremely difficult for the above-described conventional processing device and processing method to remove an extremely low concentration of EO. For example, a medical instrument that has been sterilized by ethylene oxide is stored in a vinyl booth, a heating cabinet, or the like for a predetermined period, and EO remaining in the sterilized medical instrument is emitted. In this case, it is extremely difficult for the above-described conventional processing device and processing method to remove an extremely low concentration of EO such as EO emitted into a limited space such as the vinyl booth or the heating cabinet.

Therefore, a main object of the present invention is to provide an ethylene oxide removal method and an ethylene oxide removal device that can efficiently remove even an extremely low concentration of ethylene oxide emitted into a predetermined limited space to remove ethylene oxide from the environment.

### Solution to Problem

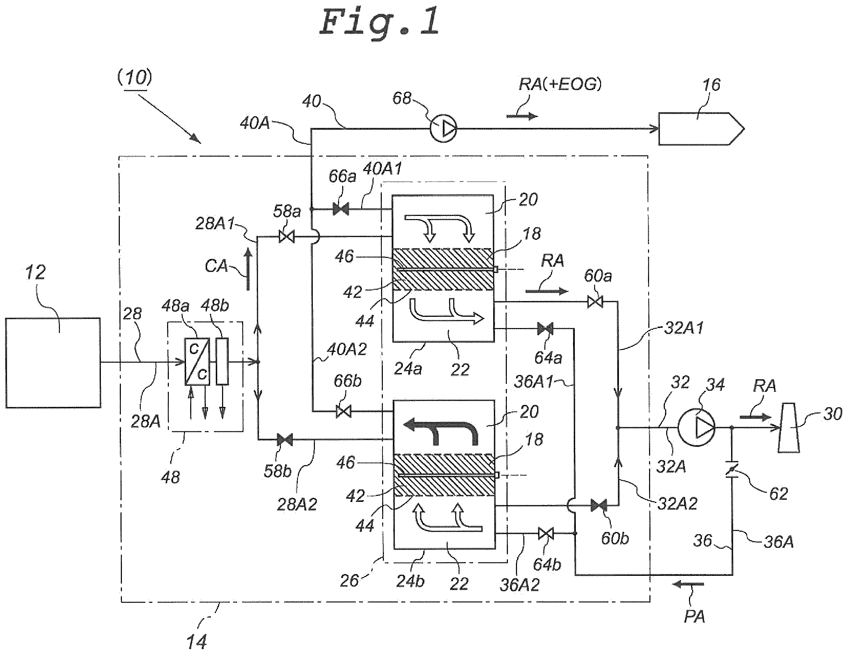

In order to attain the aforementioned object, for example, a method according to a first aspect of the present invention for removing ethylene oxide emitted into a predetermined partition space 12 is configured as follows, as shown in FIG. 1.

That is, the method includes: a first step of suctioning EO-containing air CA that contains ethylene oxide emitted into the partition space 12 and supplying the EO-containing air CA to a concentration device 14; a second step of removing a water content from the suctioned EO-containing air CA, and thereafter adsorbing and concentrating ethylene oxide by an adsorbing material 42, in the concentration device 14; a third step of desorbing the concentrated ethylene oxide from the adsorbing material 42 and sending the ethylene oxide to a removal device 16; and a fourth step of decomposing and removing ethylene oxide by the removal device 16.

According to the first aspect, for example, the following effect is exhibited.

EO in the atmosphere has characteristics similar to those of a water content. Therefore, in a case where an amount of EO emitted into the predetermined partition space 12 is small and a concentration of the EO is extremely low, it is very difficult to adsorb/remove EO from the EO-containing air CA in the partition space 12 by using the adsorbing material 42. Therefore, according to the present invention, in the second step, a water content is removed from the EO-containing air CA before EO in the EO-containing air CA is adsorbed by the adsorbing material 42. Thus, EO adsorbing efficiency of the adsorbing material 42 can be significantly enhanced.

The "predetermined partition space" refers to a closed space in which EO stays, and specifically refers to, for example, an internal space of an EO sterilizer, an internal space of a vinyl booth or a heating cabinet for temporarily stocking equipment involved in emission of EO remaining in a sterilized medical instrument, and an internal space of a workplace or a plant in which EO is handled.

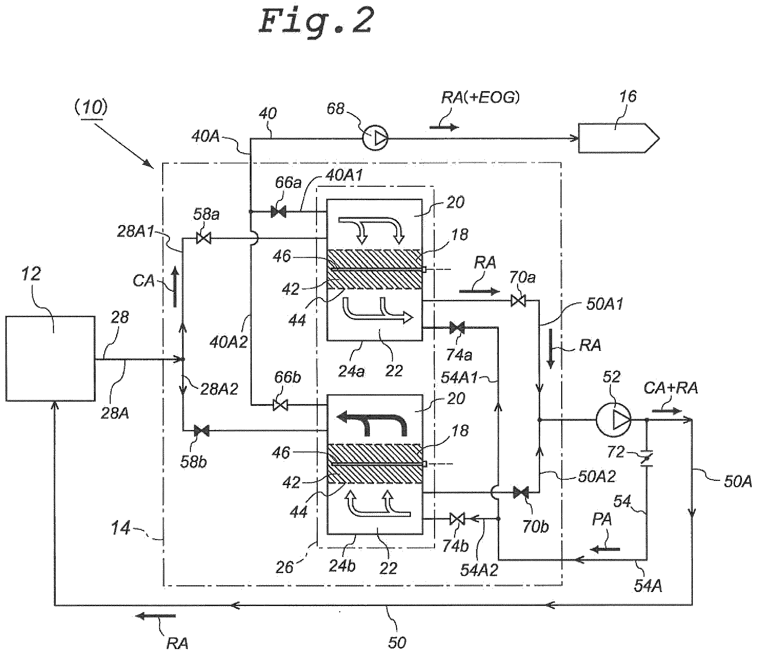

According to the present invention, for example, as shown in FIG. 2, it is preferable that, in the second step, hydrophilic zeolite is used as the adsorbing material 42, and regenerated air RA passing through the concentration device 14 is returned into the partition space 12 and circulated. By using hydrophilic zeolite as the adsorbing material 42, after or while a water content in EO-containing air CA is removed by the adsorbing material, ethylene oxide can be adsorbed. Therefore, the concentration device 14 need not have water content removal means (for example, water content removal means 48) other than the adsorbing material 42, and the device configuration can be simplified. In the regenerated air RA having passed through the concentration device 14, a content of water is low. Therefore, by returning the regenerated air RA into the predetermined partition space, a water content of the EO-containing air CA in the predetermined partition space 12 can be reduced. According to these effects, a large amount of the EO-containing air CA having an extremely low concentration of ethylene oxide can also be processed.

A device according to a second aspect is directed to an ethylene oxide gas removal system for performing the above-described method (first aspect), and, for example, includes a concentration device 14 configured to concentrate ethylene oxide emitted into a predetermined partition space 12; and a removal device 16 configured to decompose and remove ethylene oxide gas concentrated by the concentration device 14, as shown in FIG. 1.

The concentration device 14 includes: a stationary adsorption tower 26 having at least two adsorbers 24a, 24b ... in each of which an internal space is partitioned into a first chamber 20 and a second chamber 22 through an adsorption structure 18; a partition space air supply flow path 28 having an upstream end connected to the partition space 12 and having downstream ends connected to the first chambers 20 of the adsorbers 24a, 24b ..., the partition space air supply flow path 28 configured to supply EO-containing air CA supplied from the partition space 12 to any of the first chambers 20 of the adsorbers 24a, 24b ... by performing switching thereamong; a regenerated air supply flow path 32 having upstream ends connected to the second chambers 22 of the adsorbers 24a, 24b ... and having a downstream end connected to an outlet 30 that communicates with outside air, the regenerated air supply flow path 32 configured to supply, to the outlet 30, regenerated air RA obtained by adsorbing away and removing ethylene oxide through the adsorption structure 18 of any of the adsorbers 24a, 24b ...; a purge air supply flow path 36 having an upstream end connected to the regenerated air supply flow path 32 on an outlet side of a gas sending fan 34 disposed in the regenerated air supply flow path 32 and having downstream ends connected to the second chambers 22 of the adsorbers 24a, 24b ..., the purge air supply flow path 36 configured to supply a part of the regenerated air RA as purge air PA to any of the second chambers 22 of the adsorbers 24a, 24b ... by performing switching thereamong; and a concentrated EO discharge flow path 40 having upstream ends connected to the first chambers 20 of the adsorbers 24a, 24b ... and having a downstream end connected to the removal device 16. The adsorption structure 18 includes a granular or agglomerate adsorbing material 42 that is mainly formed of an inorganic porous material and physically adsorbs ethylene oxide in air, a gas-permeable casing 44 that stores the adsorbing material 42 and partitions the internal space of a corresponding one of the adsorbers 24a, 24b ... into the two chambers 20, 22 between which gas can flow, and a heating unit 46 that is embedded in the adsorbing material 42 stored in the casing 44 to directly heat the adsorbing material 42. A water content removal unit 48 that removes a water content in the EO-containing air CA is disposed preceding the stationary adsorption tower 26, in the partition space air supply flow path 28.

According to the second aspect, for example, the following effect is exhibited.

Before EO in the EO-containing air CA is adsorbed by the adsorbing material 42, a water content in the EO-containing air CA is removed by the water content removal unit 48. Therefore, EO adsorbing efficiency of the adsorbing material 42 can be significantly enhanced.

The stationary adsorption tower 26 includes at least two adsorbers 24a, 24b ..... Therefore, the two different process steps, that is, a step of adsorbing EO from the EO-containing air CA by the adsorbing material 42 and a step of regenerating (desorbing EO) and cooling the adsorbing material 42, can be simultaneously progressed merely by switching between air flow paths or on/off operation of the heating unit 46. Therefore, the ethylene oxide gas removal system can be continuously operated for a long time period.

Furthermore, a device according to a third aspect is directed to an ethylene oxide gas removal system for performing the above-described method (first aspect), and, for example, includes a concentration device 14 configured to concentrate ethylene oxide emitted into a predetermined partition space 12; and a removal device 16 configured to decompose and remove ethylene oxide concentrated by the concentration device 14, as shown in FIG. 2.

The concentration device 14 includes: a stationary adsorption tower 26 having at least two adsorbers 24a, 24b ... in each of which an internal space is partitioned into a first chamber 20 and a second chamber 22 through an adsorption structure 18; a partition space air supply flow path 28 having an upstream end connected to the partition space 12 and having downstream ends connected to the first chambers 20 of the adsorbers 24a, 24b ..., the partition space air supply flow path 28 configured to supply EO-containing air CA supplied from the partition space 12 to any of the first chambers 20 of the adsorbers 24a, 24b ... by performing switching thereamong; a regenerated air return flow path 50 having upstream ends connected to the second chambers 22 of the adsorbers 24a, 24b... and having a downstream end connected to the partition space 12, the regenerated air return flow path 50 configured to return, to the partition space 12, regenerated air RA obtained by adsorbing away and removing ethylene oxide through the adsorption structure 18 of any of the adsorbers 24a, 24b. a purge air supply flow path 54 having an upstream end connected to the regenerated air return flow path 50 on an outlet side of a gas sending fan 52 disposed in the regenerated air return flow path 50 and having downstream ends connected to the second chambers 22 of the adsorbers 24a, 24b ..., the purge air supply flow path 54 configured to supply a part of the regenerated air RA as purge air PA to any of the second chambers 22 of the adsorbers 24a, 24b ... by performing switching thereamong; and a concentrated EO discharge flow path 40 having upstream ends connected to the first chambers 20 of the adsorbers 24a, 24b ... and having a downstream end connected to the removal device 16. The adsorption structure 18 includes a granular or agglomerate adsorbing material 42 that is formed of hydrophilic zeolite and physically adsorbs a water content and ethylene oxide gas in the EO-containing air CA, a gas-permeable casing 44 that stores the adsorbing material 42 and partitions the internal space of a corresponding one of the adsorbers 24a, 24b ... into the two chambers 20, 22 between which gas can flow, and a heating unit 46 that is embedded in the adsorbing material 42 stored in the casing 44 to directly heat the adsorbing material 42.

According to the third aspect, for example, the following effect is exhibited.

Since the concentration device 14 does not have water content removal means other than the adsorbing material 42, the device configuration can be simplified, and, furthermore, the ethylene oxide gas removal system becomes suitable for processing a large amount of air.

The stationary adsorption tower 26 includes at least two adsorbers 24a, 24b .... Therefore, the two different process steps, that is, a step of adsorbing EO from the EO-containing air CA by the adsorbing material 42 and a step of regenerating (desorbing EO) and cooling the adsorbing material 42, can be simultaneously progressed merely by switching between air flow paths or on/off operation of the heating unit 46. Therefore, the ethylene oxide gas removal system can be continuously operated for a long time period.

### Advantageous Effects of Invention

The present invention can provide the ethylene oxide gas removal method and the ethylene oxide gas removal device that can efficiently remove an extremely low concentration of ethylene oxide gas emitted into a predetermined partition section to remove ethylene oxide gas from the environment.

### Brief Description of Drawings

[FIG. 1] FIG. 1 is a schematic flow diagram illustrating an example of an ethylene oxide gas removal system according to one embodiment (first embodiment) of the present invention, and illustrates a state where an adsorber 24a at an upper stage adsorbs EO, and an adsorber 24b at a lower stage regenerates and cools an adsorbing material 42.
[FIG. 2] FIG. 2 is a schematic flow diagram illustrating an example of an ethylene oxide gas removal system according to another embodiment (second embodiment) of the present invention, and illustrates a state where an adsorber 24a at an upper stage adsorbs EO, and an adsorber 24b at a lower stage regenerates and cools an adsorbing material 42.

### Description of Embodiments

Preferred embodiments of an ethylene oxide gas removal method and an ethylene oxide gas removal system using the method according to the present invention will be described below with reference to the drawings.

FIG. 1 is a schematic flow diagram illustrating an example of an ethylene oxide gas removal system 10 according to one embodiment (first embodiment) of the present invention. The ethylene oxide gas removal system 10 is a device that concentrates and collects EO existing in a predetermined partition space 12 and thereafter decomposes and removes EO, and mainly includes a concentration device 14 and a removal device 16.

The predetermined partition space 12 refers to a closed space in which EO stays as described above. The present embodiment will be described by using, as an example, an internal space of a vinyl booth or a heating cabinet for temporarily stocking equipment involved in emission of EO remaining in a sterilized medical instrument.

The concentration device 14 is a device that takes in EO from EO-containing air CA supplied from the partition space 12 and concentrates EO, and mainly includes a stationary adsorption tower 26, a partition space air supply flow path 28, a regenerated air supply flow path 32, a purge air supply flow path 36, and a concentrated EO discharge flow path 40.

The stationary adsorption tower 26 is a device that adsorbs EO in the EO-containing air CA supplied from the partition space 12 through the partition space air supply flow path 28, and includes two (in the illustrated embodiment) adsorbers 24a, 24b in each of which an internal space is partitioned into a first chamber 20 and a second chamber 22 through an adsorption structure 18.

The adsorption structure 18 includes a granular or agglomerate adsorbing material 42 that is mainly formed of an inorganic porous material and physically adsorbs EO in the air, a gas-permeable casing 44 that stores the adsorbing material 42 and partitions the internal space of the adsorber 24a, 24b into the two chambers 20 and 22 between which gas can flow, and heating means 46 that is embedded in the adsorbing material 42 stored in the casing 44 to directly heat the adsorbing material 42.

Examples of the inorganic porous material of the adsorbing material 42 include zeolite, silica gel, and activated alumina. However, zeolite is particularly suitable in consideration of, for example, ethylene oxide gas adsorbing properties and handleability.

Furthermore, in a case where zeolite is used as the adsorbing material 42, hydrophilic zeolite and hydrophobic zeolite may be mixed or disposed at multiple stages in order to make the device compact, and natural zeolite that naturally occurs, artificial zeolite that is artificially synthesized, or a mixture thereof may be used.

The hydrophilic zeolite may be A-type (Si/Al=1.0) zeolite or X-type (Si/Al=1.0 to 1.5) zeolite having a low Si/Al ratio and high hydrophilicity, or zeolite that adsorbs a larger amount of water in adsorption selectivity with respect to water and a compound, such as hydrocarbon, having a low polarity. However, the hydrophilic zeolite is synthetic zeolite that is formed of crystalline aluminosilicate and has a pore diameter of less than 6Å, and the hydrophilic zeolite preferably adsorbs at least water, more preferably can adsorb both water and ethylene oxide, and particularly preferably desorbs at least 50% of adsorbed ethylene oxide as ethylene oxide by heating or the like such that a concentration of ethylene oxide in ethylene-oxide-containing gas at the time of desorption is higher than a concentration of ethylene oxide in ethylene-oxide-containing gas at the time of adsorption since the concentration process is for enhancing combustion efficiency.

The hydrophobic zeolite may be zeolite having a high Si/Al ratio, or zeolite having a reduced surface polarity such as zeolite which has been subjected to dealuminization, zeolite in which a silanol group on the zeolite surface is modified with alkylsilane or alkoxysilane, or zeolite in which an alkyl group is modified with alcohol or the like. The hydrophobic zeolite may be hydrophobic zeolite that adsorbs a larger amount of hydrocarbon in adsorption selectivity with respect to water and a compound, such as hydrocarbon, having a low polarity. However, the hydrophobic zeolite is preferably hydrophobic zeolite that can adsorb both water and ethylene oxide.

Alternatively, in a case where the adsorbing material 42 comes into contact with gas in which the concentration of ethylene oxide and the concentration of water are equal to each other, the adsorption may be performed by using any one of the adsorbing material 42 that adsorbs a larger amount of ethylene oxide than an amount of water, the adsorbing material 42 that adsorbs a smaller amount of ethylene oxide than an amount of water, and the adsorbing material 42 that adsorbs almost the same amounts of ethylene oxide and water, the adsorption may be performed by mixing two or more of such adsorbing materials 42, or the adsorption may be performed by disposing such adsorbing materials 42 at multiple stages. In a case where the adsorbing materials 42 are disposed substantially at multiple stages, for example, in a case where ethylene oxide is adsorbed after a large amount of water is adsorbed, an inner portion of a separation device need not be partitioned by a frame or the like.

Particularly, in a case where ethylene oxide is adsorbed and removed by the adsorbing material 42 that performs physical adsorption, water that has a higher concentration than ethylene oxide or a larger gas amount in gas than ethylene oxide is adsorbed in a larger amount than ethylene oxide by the porous adsorbing material 42 that physically adsorbs ethylene oxide regardless of whether the adsorbing material 42 is hydrophilic or hydrophobic and regardless of the pore diameter, and the adsorption amount may exceed an adsorption saturation amount of the adsorbing material 42 in a short time period to cause breakthrough.

Particularly, water (2.8Å) or carbon dioxide (2.8Å) having a smaller molecular diameter than ethylene oxide (4.2Å) is likely to be adsorbed in a larger amount than ethylene oxide.

Alternatively, even zeolite having a pore diameter greater than water and less than ethylene oxide can adsorb, at its surface, ethylene oxide having a high polarity, and may be thus used to remove ethylene oxide.

The adsorbing material 42 may be an absorbing material that is mainly formed of the inorganic porous material. That is, the adsorbing material 42 may be an adsorbing material in which a content of the inorganic porous material is greater than 50 mass% with respect to the entirety of the adsorbing material 42. The entirety of the adsorbing material 42 may be formed of the inorganic porous material. Furthermore, for example, the adsorbing material 42 may contain another adsorbing material such as activated carbon and an organic adsorbing material in an amount of less than 50 mass% as necessary.

The gas-permeable casing 44 is, for example, formed of a material that does not hinder gas permeability and has excellent heat resistance and mechanical strength, and the examples of the material include metal mesh, a heat-resistant resin net, punched metal, and expanded metal.

The heating means 46 may have any form as long as the heating means is embedded in the adsorbing material 42 stored in the casing 44, and can directly heat the adsorbing material 42, more specifically, can directly heat the adsorbing material 42 itself and/or EO adsorbed in the adsorbing material 42, to desorb EO from the adsorbing material 42. As the heating means 46, an electric heater, a microwave heating device, a high-frequency induction heating device, or the like is suitably used. In the embodiment illustrated in FIG. 1, as the heating means 46, a sheathed heater in which a heating element such as a nichrome wire is mounted in a heater pipe formed as an alumina tube or a quartz tube (not shown), is embedded in an almost planar shape so as to meander in the horizontal direction. By using the heating means 46 having such a structure, the temperature can be easily controlled so as to be quickly increased over the entirety of the adsorption structure 18.

In a case where a microwave heating device is used as the heating means 46, and the casing 44 is formed of a metal, the surface of the casing 44 needs to be coated with glass, heat-resistant resin, or the like.

In the embodiment illustrated in FIG. 1 (and FIG. 2), the stationary adsorption tower 26 includes the two adsorbers 24a, 24b. However, in the stationary adsorption tower 26, the number of the adsorbers 24a, 24b ... may be two or more, and can be selected as appropriate according to the target quality of regenerated air RA, a required amount of regenerated air RA, etc. For example, when the number of the adsorbers 24a, 24b disposed in the stationary adsorption tower 26 is two as in the illustrated embodiment, two different process steps such as adsorption of EO in the EO-containing air CA, and regeneration and cooling of the adsorbing material 42 as described below can be simultaneously progressed, and, furthermore, the adsorption device can have a minimized size and have excellent space efficiency.

Meanwhile, in a case where the number of the adsorbers 24a, 24b ... disposed in the stationary adsorption tower 26 is three or more, an amount of the EO-containing air CA to be processed can be increased, and, furthermore, pressure fluctuation, hunting, or the like can be inhibited from occurring during switching to the adsorber 24a, 24b ... which is to perform an adsorption operation. The regeneration and cooling of the adsorbing material 42 can also be separately performed as different process steps, respectively.

The adsorber 24a, 24b ... disposed in the stationary adsorption tower 26 is preferably formed such that the internal space is divided into two portions in the height direction by the adsorption structure 18, and the upper side portion is the first chamber 20 and the lower side portion is the second chamber 222 as shown in FIG. 1. Thus, gas such as purge air PA having a high temperature in the adsorber 24a, 24b ... enters the adsorber 24a, 24b .... from the lower side and flows upward. Therefore, gas smoothly flows with efficiency, leading to reduction of running cost.

The partition space air supply flow path 28 is a flow path for supplying the EO-containing air CA from the partition space 12 to the stationary adsorption tower 26, and has a pipe path 28A having an upstream end connected to the partition space 12. The pipe path 28A is, for example, formed of a metal material or the like such as a stainless pipe, and is branched partway into a plurality of branched pipes 28A1, 28A2 ... (two branched pipes 28A1 and 28A2 in the illustrated embodiment), and the downstream ends of the branched pipes are connected to the first chambers 20 of the adsorbers 24a, 24b, respectively. Water content removal means 48 described below is disposed partway (before the pipe path 28A is branched) in the pipe path 28A. The branched pipes 28A1, 28A2 ... have valves 58a, 58b ..., respectively, mounted thereto. By opening and closing the valves 58a, 58b ..., a destination to which the EO-containing air CA is supplied is switched. As the valves 58a, 58b ..., for example, not only standard valves such as butterfly valves and ball valves but also dampers or the like can be used (hereinafter, the same applies to all valves.)

The water content removal means 48 is a device for removing a water content from the EO-containing air CA taken out from the partition space 12, and includes a chiller 48a and a mist separator 48b in the embodiment illustrated in FIG. 1. The configuration of the water content removal means 48 is not limited to the above-described configuration, and may be any configuration that can remove a water content in the EO-containing air CA, and the water content removal means 48 may be, for example, implemented by a silica gel column.

The regenerated air supply flow path 32 is a flow path for supplying, to an outlet 30, regenerated air RA obtained by adsorbing away and removing EO through the adsorption structure 18 of either of the adsorbers 24a and 24b, and the regenerated air supply flow path 32 has a pipe path 32A having a downstream end connected to the outlet 30. The pipe path 32A is, for example, formed of a metal material or the like such as a stainless pipe, and is branched partway into a plurality of branched pipes 32A1, 32A2 ... (two branched pipes 32A1 and 32A2 in the illustrated embodiment), and the upstream ends of the branched pipes are connected to the second chambers 22 of the adsorbers 24a, 24b, respectively. A gas sending fan 34 for suctioning the regenerated air RA and sending the regenerated air RA to the outlet 30 is mounted partway in the pipe path 32A. The branched pipes 32A1, 32A2 ... have valves 60a, 60b ..., respectively, mounted thereto.

The purge air supply flow path 36 has a pipe path 36A in which an upstream end is connected to the regenerated air supply flow path 32 on an outlet side of the gas sending fan 34 disposed in the regenerated air supply flow path 32. The pipe path 36A is, for example, formed of a metal material or the like such as a stainless pipe. A butterfly valve 62 for regulating an amount of the regenerated air RA flowing into the pipe path 36A is disposed on the upstream end side, the downstream side portion of the pipe path 36A is branched into branched pipes 36A1, 36A2 ... (two branched pipes 36A1 and 36A2 in the illustrated embodiment), and the branched pipes are connected to the second chambers 22 of the adsorbers 24a, 24b ..., respectively. The branched pipes 36A1, 36A2 ... have valves 64a, 64b ..., respectively, mounted thereto. Therefore, the purge air supply flow path 36 takes in a part of the regenerated air RA as the purge air PA, and can supply the purge air PA to any of the second chambers 22 of the adsorbers 24a, 24b ... by performing switching thereamong.

The concentrated EO discharge flow path 40 is for supplying EO that is desorbed from the adsorbing material 42 and has a relatively high concentration, to the removal device 16, after the EO has been concentrated by the adsorbing material 42 of the adsorption structure 18, through the purge air PA supplied to the adsorber 24a, 24b .... The concentrated EO discharge flow path 40 has a pipe path 40A through which the purge air PA including the concentrated EO flows. The pipe path 34A is, for example, formed of a metal material or the like such as a stainless pipe, and the upstream side portion thereof is branched into branched pipes 40A1, 40A2 ... (two branched pipes 40A1 and 40A2 in the illustrated embodiment), and the branched pipes are connected to the first chambers 20 of the adsorbers 24a, 24b ..., respectively. The branched pipes 40A1, 40A2 ... have valves 66a, 66b ..., respectively, mounted thereto. A gas sending fan 68 for suctioning the purge air PA including the concentrated EO and supplying the purge air PA to the removal device 16 is mounted on the downstream side of the pipe path 40A. The downstream end of the pipe path 40A is connected to the removal device 16.

The removal device 16 is a device for decomposing and removing EO concentrated by the concentration device 14. As the EO decomposition method performed by the removal device 16, a known method such as direct combustion, catalytic combustion, photocatalysis, plasma, acid/base hydrolysis, or a scrubber can be adopted. Among them, a catalytic combustion-type abator is suitably used for the removal device 16 from the viewpoint of, for example, reducing waste.

The valves, the heating means 46, the gas sending fan 34, the gas sending fan 68, and the like of the ethylene oxide gas removal system 10 having the above-described configuration are connected to not-illustrated control means, and are controlled by the control means so as to perform predetermined operations.

When EO in the partition space 12 is removed by using the ethylene oxide gas removal system 10 having the above-described configuration, at least one of the adsorbers 24a, 24b ... adsorbs EO to generate the regenerated air RA, and at least one of the adsorbers 24a, 24b ... regenerates (desorbs EO) and cools the adsorbing material 42 thereinside. For example, in the ethylene oxide gas removal system 10 of the embodiment illustrated in FIG. 1, the upper-stage adsorber 24a removes EO in the EO-containing air CA to generate the regenerated air RA, and the lower-stage adsorber 24b regenerates (desorbs EO adsorbed in the previous operation) and cools the adsorbing material 42. The method will be specifically described below.

As shown in FIG. 1, the valve 58a of the partition space air supply flow path 28 is opened and the valve 58b thereof is closed. The valve 60a of the regenerated air supply flow path 32 is opened, and the valve 60b thereof is closed. Furthermore, the valve 64b of the purge air supply flow path 36 and the valve 66b of the concentrated EO discharge flow path 40 are opened, the valve 64a of the purge air supply flow path 36 and the valve 66a of the concentrated EO discharge flow path 40 are closed, and the heating means 46 of the adsorber 24b is operated. When the temperature of the heating means 46 of the adsorber 24b has reached a predetermined temperature (for example, a predetermined temperature of 180°C or higher in the case of the adsorbing material 42 being zeolite) at which EO is desorbed from the adsorbing material 42, operations of the gas sending fan 34 and the gas sending fan 68 are started. Then, a first step (in the ethylene oxide gas removal method) of suctioning the EO-containing air CA from the partition space 12 and supplying the EO-containing air CA to the concentration device 14 is performed.

Subsequently, the EO-containing air CA passes through the water content removal means 48 while flowing toward the first chamber 20 of the adsorber 24a. At this time, a water content in the EO-containing air CA is removed. Thereafter, when the EO-containing air CA from which the water content has been removed and which has reached the first chamber 20 of the adsorber 24a passes through the adsorption structure 18, EO is adsorbed by the adsorbing material 42. When the EO-containing air CA reaches the second chamber 22, the EO-containing air CA has changed to the regenerated air RA from which EO has been removed, thereby completing a second step (in the ethylene oxide gas removal method).

Subsequently, the regenerated air RA is suctioned by the gas sending fan 34, sent to the outlet 30 through the regenerated air supply flow path 32, and emitted into the atmosphere through the outlet 30. A part of the regenerated air RA is sent to the purge air supply flow path 36, and used as the purge air PA.

The purge air PA is sequentially sent to the second chamber 22 of the adsorber 24b through the branched pipe 36A2. EO adsorbed and concentrated by the adsorbing material 42 is desorbed from the adsorbing material 42 by the purge air PA that has reached the second chamber 22 of the adsorber 24b when the purge air PA passes through the adsorption structure 18, and both the purge air PA and the desorbed EO reach the first chamber of the adsorber 24b, thereby completing a third step (in the ethylene oxide gas removal method).

When regeneration of the adsorbing material 42, that is, desorption of EO adsorbed by the adsorbing material 42 of the adsorber 24b has been completed, the operation of the heating means 46 of the adsorber 24b is halted. Then, cooling of the adsorbing material 42 by the purge air PA flowing through the adsorber 24b at a normal temperature is started.

Both the purge air PA that reaches the first chamber 20 of the adsorber 24b and EO concentrated at a high concentration, are suctioned by the gas sending fan 68 and supplied through the concentrated EO discharge flow path 40 to the removal device 16, and EO is decomposed and removed by the removal device 16. That is, a fourth step (in the ethylene oxide gas removal method) is performed.

In a case where, in the above-described state, the ethylene oxide gas removal system 10 is operated over a predetermined time, an amount of EO adsorbed by the adsorbing material 42 of the adsorber 24a approaches a saturated amount. Then, switching between the adsorbers 24a and 24b needs to be performed. That is, in the ethylene oxide gas removal system 10 in the state shown in FIG. 1, the valve 58b of the partition space air supply flow path 28 is opened, and the valve 58a thereof is closed. The valve 60b of the regenerated air supply flow path 32 is opened, and the valve 60a thereof is closed. Furthermore, the valve 64a of the purge air supply flow path 36 and the valve 66a of the concentrated EO discharge flow path 40 are opened, the valve 64b of the purge air supply flow path 36 and the valve 66b of the concentrated EO discharge flow path 40 are closed, and the heating means 46 of the adsorber 24a is operated. Thus, the upper-stage adsorber 24a regenerates the adsorbing material 42 and the lower-stage adsorber 24b adsorbs EO.

Thereafter, the switching between the valves and the on/off operation of each heating means 46 are sequentially performed, and switching of the operation between the adsorbers 24a and 24b is sequentially performed.

In the ethylene oxide gas removal system 10 of the present embodiment, before EO in the EO-containing air CA is adsorbed by the adsorbing material 42, the water content removal means 48 removes a water content in the EO-containing air CA, so that EO adsorbing efficiency of the adsorbing material 42 can be significantly enhanced. Specifically, in a case where the partition space 12 is a vinyl booth or a heating cabinet for temporarily stocking a medical instrument having been subjected to EO sterilization, the EO concentration in the EO-containing air CA ranges from 5 to 1000 ppm in general whereas the EO concentration in the regenerated air RA is less than 1 ppm.

Since the concentration device 14 is disposed outside the partition space 12, the devices of the ethylene oxide gas removal system 10 can be collectively disposed near each other, and the EO removal efficiency can be enhanced.

In the above-described embodiment, the concentration device 14 merely includes one stationary adsorption tower 26. However, two or more stationary adsorption towers 26 may be disposed and connected in parallel. Thus, an amount of the EO-containing air CA that is processed per unit time can be increased.

Increase of the number of the stationary adsorption towers 26 increases the number of piping systems and also increases the number of valves according to increase of the number of the piping systems. In this case, preferably, valves having the same function are collectively stored in a duct, and the duct is caused to have a function as the branched pipe of each pipe path (the illustration is omitted).

In the above-described embodiment, the concentration device 14 is disposed outside the partition space 12. However, the concentration device 14 may be disposed inside the partition space 12. In this case, a long complex piping system can be eliminated, and the configuration can be made compact. As a result, air sending power can be reduced.

In the above-described embodiment, a sheathed heater (electric heater) is used as the heating means 46 of the adsorption structure 18. However, the heating means 46 may be implemented by any means that is embedded in the adsorbing material 42 stored in the casing 44, to directly heat the adsorbing material 42. Therefore, for example, exhaust gas from an abator used as the removal device 16 is introduced into the casing 44, and heat (exhaust heat) of the exhaust gas may be used as a heat source of the heating means 46. In this case, power used for heating the adsorbing material 42 can be eliminated or reduced.

Next, the ethylene oxide gas removal system 10 of a second embodiment illustrated in FIG. 2 will be described.

The ethylene oxide gas removal system 10 of the second embodiment is different from the ethylene oxide gas removal system 10 of the above-described first embodiment in that the adsorbing material 42 is limited to a material formed of hydrophilic zeolite, and a regenerated air return flow path 50 is disposed instead of the regenerated air supply flow path 32, and the regenerated air RA is returned to the partition space 12 and circulated, in the second embodiment. The components other than these components are the same as those of the first embodiment. Therefore, the same components are denoted by the same reference characters (reference signs) as in the first embodiment, and the description of the first embodiment is referred to without repeatedly describing the components.

Hydrophilic zeolite used as the adsorbing material 42 is synthetic zeolite that is formed of crystalline aluminosilicate and has a pore diameter of less than 6Å, and can adsorb both a water content and ethylene oxide. The shape of the adsorbing material 42 formed of the hydrophilic zeolite is not particularly limited as long as the adsorbing material 42 is granular or agglomerate. However, the adsorbing material 42 preferably has a pellet-like shape having a pellet diameter of 2 to 3 mm and a pellet length of about 4 to 6 mm in consideration of EO adsorbing efficiency and the like.

The regenerated air return flow path 50 is a flow path for returning the regenerated air RA obtained by adsorbing away and removing EO through the adsorption structure 18 of any of the adsorbers 24a, 24b, to the partition space 12, to circulate the regenerated air RA, and has a pipe path 50A having a downstream end connected to the partition space 12. The pipe path 50A is, for example, formed of a metal material or the like such as a stainless pipe, and is branched partway into a plurality of branched pipes 50A1, 50A2 ... (two branched pipes 50A1 and 50A2 in the illustrated embodiment), and the upstream ends of the branched pipes are connected to the second chambers 22 of the adsorbers 24a, 24b, respectively. A gas sending fan 52 for suctioning the regenerated air RA and supplying the regenerated air RA to the partition space 12 is disposed partway in the pipe path 50A. The branched pipes 50A1, 50A2 ... have valves 70a, 70b ..., respectively, mounted thereto.

A purge air supply flow path 54 has a pipe path 54A having an upstream end connected to the regenerated air return flow path 50 on the outlet side of the gas sending fan 52 disposed in the regenerated air return flow path 50. The pipe path 54A is, for example, formed of a metal material or the like such as a stainless pipe. A butterfly valve 72 for regulating an amount of the regenerated air RA flowing into the pipe path 54A is disposed on the upstream end side of the pipe path 54A. The downstream side portion of the pipe path 54A is branched into branched pipes 54A1, 54A2 ... (two branched pipes 54A1 and 54A2 in the illustrated embodiment), and the branched pipes are connected to the second chambers 22 of the adsorbers 24a, 24b ..., respectively. The branched pipes 54A1, 54A2 ... have valves 74a, 74b ..., respectively, mounted thereto. Therefore, the purge air supply flow path 54 takes in a part of the regenerated air RA as the purge air PA, and can supply the purge air PA to any of the second chambers 22 of the adsorbers 24a, 24b ... by performing switching thereamong.

Next, when EO in the partition space 12 is removed by using the ethylene oxide gas removal system 10 of the second embodiment having the above-described configuration, at least one of the adsorbers 24a, 24b ... adsorbs EO to generate the regenerated air RA, and at least one of the adsorbers 24a, 24b ... regenerates (desorbs EO) and cools the adsorbing material 42 thereinside. For example, in the embodiment illustrated in FIG. 2, the upper-stage adsorber 24a removes EO in the EO-containing air CA to generate the regenerated air RA, and the lower-stage adsorber 24b regenerates (desorbs EO adsorbed in the previous operation) and cools the adsorbing material 42. The method will be specifically described below.

As shown in FIG. 2, the valve 58a of the partition space air supply flow path 28 is opened, and the valve 58b thereof is closed. The valve 70a of the regenerated air return flow path 50 is opened, and the valve 70b thereof is closed. Furthermore, the valve 74b of the purge air supply flow path 54 and the valve 66b of the concentrated EO discharge flow path 40 are opened, the valve 74a of the purge air supply flow path 54 and the valve 66a of the concentrated EO discharge flow path 40 are closed, and the heating means 46 of the adsorber 24b is operated. When the temperature of the heating means 46 of the adsorber 24b has reached a predetermined temperature (predetermined temperature of 180°C or higher since the adsorbing material 42 is hydrophilic zeolite) at which EO is desorbed from the adsorbing material 42, operations of the gas sending fan 52 and the gas sending fan 68 are started. Then, the EO-containing air CA is suctioned from the partition space 12 and supplied to the concentration device 14.

Subsequently, when the EO-containing air CA having reached the first chamber 20 of the adsorber 24a in the concentration device 14 passes through the adsorption structure 18, water and EO in the EO-containing air CA are adsorbed by the adsorbing material 42. However, since a water content (2,000 to 20,000 ppm) is substantially greater than an amount of EO, the water content is selectively adsorbed (earlier than adsorption of EO). Therefore, a lot of the EO-containing air CA having reached the second chamber 22 is not cleansed so as to generate the regenerated air RA, and is mainly changed to the EO-containing air CA in which a water content is reduced. The EO-containing air CA in which a water content is reduced is suctioned by the gas sending fan 52, and returned through the regenerated air return flow path 50 to the partition space 12. Such a circulation is repeated. When a water content of the EO-containing air CA is reduced to a certain or lower level, an amount of EO adsorbed by the adsorbing material 42 of the adsorber 24a is increased, the EO-containing air CA is cleansed to generate the regenerated air RA, and the EO concentration in the partition space 12 is gradually reduced. In a case where the adsorption structure 18 has a necessary and sufficient amount of the adsorbing material 42, a water content and EO can be adsorbed and removed from the EO-containing air CA, and the EO-containing air CA can be cleansed to generate the regenerated air RA by causing the EO-containing air CA to pass through the adsorbing material once.

Subsequently, the regenerated air RA is returned to the partition space 12 and circulated as described above. A part of the regenerated air RA is sent to the purge air supply flow path 54 and used as the purge air PA.

The purge air PA is sequentially sent through the branched pipe 54A2 to the second chamber 22 of the adsorber 24b. EO adsorbed and concentrated by the adsorbing material 42 is desorbed from the adsorbing material 42 by the purge air PA that has reached the second chamber 22 of the adsorber 24b when the purge air PA passes through the adsorption structure 18, and both the purge air PA and the desorbed EO reach the first chamber of the adsorber 24b.

When the adsorbing material 42 has been regenerated, that is, EO adsorbed by the adsorbing material 42 of the adsorber 24b has been desorbed, operation of the heating means 46 of a deodorizing unit 24b is halted. Thus, cooling of the adsorbing material 42 by the purge air PA flowing through the adsorber 24b at a normal temperature is started.

Both the purge air PA that reaches the first chamber 20 of the adsorber 24b and EO concentrated at a high concentration, are suctioned by the gas sending fan 68 and supplied through the concentrated EO discharge flow path 40 to the removal device 16, and EO is decomposed and removed by the removal device 16.

In the ethylene oxide gas removal system 10 of the present embodiment, since the concentration device 14 does not have water content removal means other than the adsorbing material 42, the device configuration can be made simple and compact, and, furthermore, the ethylene oxide gas removal system 10 becomes suitable for processing a large amount of air.

In the above-described embodiment, the concentration device 14 merely includes one stationary adsorption tower 26. However, as described above for the first embodiment, two or more stationary adsorption towers 26 may be disposed and connected in parallel. Thus, an amount of the EO-containing air CA that is processed can be increased.

The concentration device 14 is disposed outside the partition space 12. However, as described above for the first embodiment, the concentration device 14 may be disposed inside the partition space 12.

It is needless to say that various other modifications can be made within the scope conceived by persons skilled in the art.

### Reference Signs List

- 10: ethylene oxide gas removal system
- 12: partition space
- 14: concentration device
- 16: removal device
- 18: adsorption structure
- 20: first chamber
- 22: second chamber
- 24a, 24b: adsorber
- 26: stationary adsorption tower
- 28: partition space air supply flow path
- 30: outlet
- 32: regenerated air supply flow path
- 34: gas sending fan
- 36: purge air supply flow path
- 40: concentrated EO discharge flow path
- 42: adsorbing material
- 44: casing
- 46: heating means
- 48: water content removal means
- 50: regenerated air return flow path
- 52: gas sending fan
- 54: purge air supply flow path
- CA: EO-containing air
- RA: regenerated air
- PA: purge air

## Claims

1. A method for removing ethylene oxide gas emitted into a predetermined partition space (12), the method comprising:
a first step of suctioning EO-containing air (CA) that contains ethylene oxide emitted into the partition space (12) and supplying the EO-containing air (CA) to a concentration device (14);
a second step of removing a water content from the suctioned EO-containing air (CA), and thereafter adsorbing and concentrating ethylene oxide by an adsorbing material (42), in the concentration device (14);
a third step of desorbing the concentrated ethylene oxide from the adsorbing material (42) and sending the ethylene oxide to a removal device (16); and
a fourth step of decomposing and removing ethylene oxide by the removal device (16).

2. The ethylene oxide gas removal method according to claim 1, wherein, in the second step, hydrophilic zeolite is used as the adsorbing material (42), and regenerated air (RA) passing through the concentration device (14) is returned into the partition space (12) and circulated.

3. The ethylene oxide gas removal method according to claim 1 or 2, wherein an amount of ethylene oxide remaining in the regenerated air (RA) is less than 1 ppm after ethylene oxide is decomposed and removed.

4. An ethylene oxide gas removal system comprising:
a concentration device (14) configured to concentrate ethylene oxide gas emitted into a predetermined partition space (12); and
a removal device (16) configured to decompose and remove ethylene oxide gas concentrated by the concentration device (14), wherein
the concentration device (14) includes:
a stationary adsorption tower (26) having at least two adsorbers (24a, 24b ...) in each of which an internal space is partitioned into a first chamber (20) and a second chamber (22) through an adsorption structure (18);
a partition space air supply flow path (28) having an upstream end connected to the partition space (12) and having downstream ends connected to the first chambers (20) of the adsorbers (24a, 24b ...), the partition space air supply flow path (28) configured to supply EO-containing air (CA) supplied from the partition space (12) to any of the first chambers (20) of the adsorbers (24a, 24b ...) by performing switching thereamong;
a regenerated air supply flow path (32) having upstream ends connected to the second chambers (22) of the adsorbers (24a, 24b ...) and having a downstream end connected to an outlet (30) that communicates with outside air, the regenerated air supply flow path (32) configured to supply, to the outlet (30), regenerated air (RA) obtained by adsorbing away and removing ethylene oxide through the adsorption structure (18) of any of the adsorbers (24a, 24b ...);
a purge air supply flow path (36) having an upstream end connected to the regenerated air supply flow path (32) on an outlet side of a gas sending fan (34) disposed in the regenerated air supply flow path (32) and having downstream ends connected to the second chambers (22) of the adsorbers (24a, 24b ...), the purge air supply flow path (36) configured to supply a part of the regenerated air (RA) as purge air (PA) to any of the second chambers (22) of the adsorbers (24a, 24b ...) by performing switching thereamong; and
a concentrated EO discharge flow path (40) having upstream ends connected to the first chambers (20) of the adsorbers (24a, 24b ...) and having a downstream end connected to the removal device (16),
the adsorption structure (18) includes a granular or agglomerate adsorbing material (42) that is mainly formed of an inorganic porous material and physically adsorbs ethylene oxide in air, a gas-permeable casing (44) that stores the adsorbing material (42) and partitions the internal space of a corresponding one of the adsorbers (24a, 24b ...) into the two chambers (20, 22) between which gas can flow, and a heating unit (46) that is embedded in the adsorbing material (42) stored in the casing (44) to directly heat the adsorbing material (42), and
a water content removal unit (48) that removes a water content in the EO-containing air (CA) is disposed preceding the stationary adsorption tower (26), in the partition space air supply flow path (28).

5. An ethylene oxide gas removal system comprising:
a concentration device (14) configured to concentrate ethylene oxide gas emitted into a predetermined partition space (12); and
a removal device (16) configured to decompose and remove ethylene oxide gas concentrated by the concentration device (14), wherein
the concentration device (14) includes:
a stationary adsorption tower (26) having at least two adsorbers (24a, 24b ...) in each of which an internal space is partitioned into a first chamber (20) and a second chamber (22) through an adsorption structure (18);
a partition space air supply flow path (28) having an upstream end connected to the partition space (12) and having downstream ends connected to the first chambers (20) of the adsorbers (24a, 24b ...), the partition space air supply flow path (28) configured to supply EO-containing air (CA) supplied from the partition space (12) to any of the first chambers (20) of the adsorbers (24a, 24b ...) by performing switching thereamong;
a regenerated air return flow path (50) having upstream ends connected to the second chambers (22) of the adsorbers (24a, 24b...) and having a downstream end connected to the partition space (12), the regenerated air return flow path (50) configured to return, to the partition space (12), regenerated air (RA) obtained by adsorbing away and removing ethylene oxide through the adsorption structure (18) of any of the adsorbers (24a, 24b...);
a purge air supply flow path (54) having an upstream end connected to the regenerated air return flow path (50) on an outlet side of a gas sending fan (52) disposed in the regenerated air return flow path (50) and having downstream ends connected to the second chambers (22) of the adsorbers (24a, 24b ...), the purge air supply flow path (54) configured to supply a part of the regenerated air (RA) as purge air (PA) to any of the second chambers (22) of the adsorbers (24a, 24b ...) by performing switching thereamong; and
a concentrated EO discharge flow path (40) having upstream ends connected to the first chambers (20) of the adsorbers (24a, 24b ...) and having a downstream end connected to the removal device (16), and
the adsorption structure (18) includes a granular or agglomerate adsorbing material (42) that is formed of hydrophilic zeolite and physically adsorbs a water content and ethylene oxide in the EO-containing air (CA), a gas-permeable casing (44) that stores the adsorbing material (42) and partitions the internal space of a corresponding one of the adsorbers (24a, 24b ...) into the two chambers (20, 22) between which gas can flow, and a heating unit (46) that is embedded in the adsorbing material (42) stored in the casing (44) to directly heat the adsorbing material (42).
